# EUROPÄISCHE PATENTSCHRIFT

(11) **EP 2 386 215 B1**
(45) Veröffentlichungstag und Bekanntmachung des Hinweises auf die Patenterteilung: **12.03.2014**
(21) Anmeldenummer: 11164239.3
(22) Anmeldetag: 29.04.2011
(51) Int. Cl.: A43B 13/02

(54) **Schuheinlage und Schuh**
Shoe insert and shoe
Semelle intérieure de chaussure et chaussure

(30) Priorität: 12.05.2010 DE 102010028939
(43) Veröffentlichungstag der Anmeldung: 16.11.2011
(73) Patentinhaber: Helmut Röck Gmbh, 73252 Lenningen (DE)
(72) Erfinder: Schmid, Ulrich, 73277 Owen (DE)
(74) Vertreter: DREISS Patentanwälte PartG mbB

(56) Entgegenhaltungen:
- EP-A1- 2 111 770
- EP-A1- 2 442 683
- WO-A1-2005/120268
- WO-A2-2007/149429
- CH-A- 128 381
- FR-A1- 2 844 995
- US-A- 1 510 308
- US-A- 1 974 161
- US-B1- 6 247 250

## Beschreibung

Die Erfindung betrifft eine Schuheinlage und einen Schuh, und insbesondere eine Schuheinlage und einen Schuh, welche Fehlhaltungen bei Sportarten, insbesondere beim Radfahren, Schlittschuhlaufen, Ski-Langlauf, Skifahren (Alpin), Rudern oder dergleichen korrigieren und damit die Kraftübertragung verbessern.

Beim Radfahren ist der Fuß der wichtigste Kontaktpunkt mit dem Fahrrad. An der Schnittstelle Fuß-Radschuh-Cleat-Pedale wird die körperliche Leistung in Vorschub umgewandelt. Bei der Übertragung der Kraft entstehen in allen Bereichen des Fußes (oberes, unteres Sprunggelenk, Mittelfuß und Vorfuß) Torsionen, wodurch Kraft in horizontaler Ebene abgeleitet wird und für den Vorschub über die Pedale nicht mehr zur Verfügung steht. Leistung geht dadurch verloren.

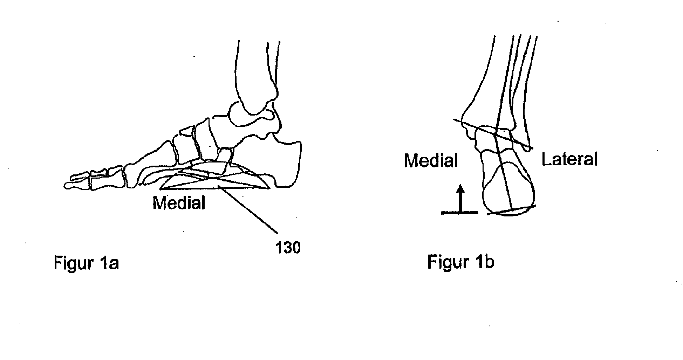

Im Einzelnen wird die produzierte Kraft der vortriebswirksamen Muskulatur (Hüftbeuger/-strecker, Beinbeuger/-strecker, Wadenmuskulatur) über die Schien- und Wadenbeinknochen auf das Sprungbein (Talus) übertragen. An dieser Stelle findet der erste größere Kraftverlust statt, da das Sprungbein nach innen knickt (Pronation) (vgl. Figur 1b). Je schwächer die Muskulatur und der Bandapparat ist, desto größer ist der Kraftreibungsverlust. Auch bei optimalen Voraussetzungen treten Torsionen auf, da die Pronation ein natürlicher Dämpfungsmechanismus ist. Beim Radfahren ist dieser Dämpfungsmechanismus jedoch nicht notwendig, da die Stoßbelastung des Körpergewichts fehlt und eine Dämpfung deshalb nicht notwendig ist.

Über die Mittelfußknochen des Fußes wird die Kraft auf das Großzehengrundgelenk weitergeleitet. Hierbei ist das Problem, dass punktuell große Kräfte auftreten, da neben dem Großzehengrundgelenk noch vier weitere Zehengelenke existieren, die nur deutlich schwächer belastet werden.

Eine entsprechende Belastung bzw. Fehlstellung des Fußes tritt auch bei anderen Sportarten auf, bei denen insbesondere mit den Füßen eine geradlinige Krafteinleitung und kein Abrollen des Fußes, wie es bei einer Gehbewegung erfolgt, stattfindet. Dies ist insbesondere beim Schlittschuhlaufen, Ski-Langlauf, Skifahren (Alpin), Rudern oder dergleichen der Fall.

Aus dem Stand der Technik sind verschiedene Schuheinlagen, insbesondere orthopädische Schuheinlagen oder Einlegesohlen bekannt. So beschreibt beispielsweise die internationale Patentanmeldung WO 02/49472 A1 eine Schuhsohle mit einer Wölbplatte, wobei die Wölbplatte im Bereich des Mittelfußes zwischen Fersenbereich und dem Ballen gewölbt werden kann.

In der internationalen Patentanmeldung WO 2007/057626 A1 wird eine inhärente Leistungsplatte beschrieben, die den Fuß durch eine Wölbung im Bereich des Talus und Os Naviculare unterstützt.

Aus der DE 20 2006 002 991 U1 und der DE 29 08 019 A1 sind Orthesen bekannt, bei denen eine natürliche Abrollbewegung des Fußes beim normalen Gehen möglich sein soll. Das Beheben einer Fehstellung bei einer geradlinigen Krafteinleitung wird nicht angesprochen.

Eine weitere Schuheinlage für Sportschuhe wird in der US-Patentschrift US 5,647,147 A beschrieben. Dabei ist vorgesehen, dass die Schuheinlage im Fersen- und Zehenbereich gegenüber dem Mittelfußknochen angehoben ist. Weitere orthopädische Schuheinlagen sind aus den Druckschriften WO 2005 120 268 A1 und FR 2 844 995 bekannt. Die US 6,247,250 B1 beschreibt Einlagen für Sportschuhe, welche keilförmige Kissen aufweisen. In der WO 2007 149 429 A2 ist eine Schuheinlage mit variierbarer Unterstützung des Längsgewölbes bekannt geworden. In der US 1,974,161 ist eine Einlage zur Verbesserung des Tragekomforts von Damenschuhen mit hohen Absätzen beschrieben. Aus der EP 2 111 770 A1 ist Schuhsohle bekannt geworden, die eine gewölbte Zone aufweist. Die Schuhsohle erstreckt sich dabei unter die Mittelfußköpfe des Fußes. Aus der US 1 510 308 A ist eine das Fußgewölbe unterstützene Sohle bekannt geworden.

Nachteilig an den bekannten Lösungen ist, dass insbesondere beim Radfahren, aber auch bei den anderen genannten Sportarten zwischen Talus und den Zehengelenken weiterhin Leistung verloren geht. Es ist daher Aufgabe der vorliegenden Erfindung, eine Schuheinlage und einen Schuh bereitzustellen, welche die Nachteile des Standes der Technik vermeiden und insbesondere die Kraftübertragung des Fußes verbessern. Insbesondere soll einem medialen Eindrehen des Fußes entgegen gewirkt werden.

Diese Aufgabe wird erfindungsgemäß durch die Merkmale in den Ansprüchen 1 und 10 gelöst. Zweckmäßige Ausgestaltungen der Erfindung sind in den Unteransprüchen enthalten.

Die zur Lösung vorgeschlagene Schuheinlage weist zunächst einen zusammenhängenden Versteifungsbereich auf, der zumindest einen Bereich der Schuheinlage umfasst, der sich bei aufliegendem Fuß im Wesentlichen vom Längsgewölbe zum Vorfuß erstreckt, wobei der Versteifungsbereich bei aufliegendem Fuß im Bereich des Längsgewölbes gegen die Fußsohle gewölbt ist, im unbelasteten Zustand sich die Bereiche der Schuheinlage, die sich bei aufliegendem Fuß im Bereich des Rückfußes und des Vorfußes befinden, im Wesentlichen in der Nullebene liegen und der Versteifungsbereich weiter so ausgebildet ist, dass bei aufgelegtem Fuß zumindest der Bereich des Großzehengrundgelenks außerhalb des Versteifungsbereichs angeordnet ist.

Dabei ist der Versteifungsbereich zur Realisierung einer Hebelwirkung derart ausgebildet, so dass bei aufgelegtem Fuß und einer Pronation des Fußes der gewölbte Bereich unter dem Längsgewölbe niedergedrückt wird und dies zu einer Anhebung des Versteifungsbereichs unter dem Vorfuß führt.

Ein besonderer Vorteil der erfindungsgemäßen Schuheinlage besteht darin, dass die beim Tragen der Schuheinlage die Kraftübertragung des gesamten Fußes auf den Schuh und somit, beispielsweise beim Radfahren, auf das Pedal verbessert wird, aber die Einlage dennoch im Schuh nur einen geringen Platzbedarf benötigt. Das wird dadurch erreicht, dass erfindungsgemäß eine Schuheinlage verwendet wird, die einen zusammenhängenden Versteifungsbereich aufweist, der bei aufgelegtem Fuß zumindest vom Längsgewölbe des Fußes bis zum Vorfuß verläuft. Die Erfindung eignete sich insbesondere zur verbesserten Kraftübertragung beim Radsport; sie kann aber auch in anderen Sportarten, wie beispielsweise Schlittschuhlaufen, Ski-Langlauf, Skifahren (Alpin), Rudern oder dergleichen zur Anwendung kommen.

In einer bevorzugten Ausführungsform erstreckt sich der Versteifungsbereich nur bis unter die Mittelfußköpfchen II bis V, oder nur bis unter die Mittelfußköpfchen III bis V, nicht jedoch bis unter die Zehen. Außerdem weist der Versteifungsbereich eine Wölbung auf, die bei aufgelegtem Fuß gegen das Längsgewölbe des Fußes gewölbt ist. In unbelastetem Zustand liegen die Bereiche der Schuheinlage, die sich bei aufliegendem Fuß im Bereich des Rückfußes und des Vorfußes, insbesondere hinter dem Längsgewölbe und unter den Mittelfußköpfchen, befinden zumindest im wesentlichen in der Nullebene, d.h. in der Ebene des Standbodens des nackten Fußes. Erfindungsgemäß ist der Versteifungsbereich weiter so ausgebildet, dass das Großzehengrundgelenk nicht auf dem Versteifungsbereich liegt. Vielmehr kommt das Großzehengrundgelenk außerhalb des Versteifungsbereichs zu liegen. Der Versteifungsbereich bildet somit einen zusammenhängenden Bereich, der im wesentlichen diagonal vom medialen Rand des Längsgewölbes bis zu den Mittelfußköpfchen II bis V bzw. bis zu den Mittelfußköpfchen III bis V verläuft. Hierdurch wird die oben erwähnte Pronation ausgenutzt, um die Kraftübertragung vom Bewegungsapparat auf den Schuh und den Untergrund bzw. das Pedal zu übertragen. Durch die Pronation wird nämlich der gewölbte Bereich unter dem Längsgewölbe niedergedrückt, was zu einer Anhebung des Versteifungsbereichs unter dem Vorfuß, insbesondere unter dem 3. bis 5. Strahl führt. Das bewirkt eine Schrägstellung des Vorfußes, wodurch der laterale Bereich des Vorfußes, insbesondere die Mittelfußköpfchen II bis V bzw. III bis V, angehoben werden. Durch die Belastung der Schuheinlage wird der Vorfuß nach medial in eine Torsionsstellung gekippt. Je nach Krafteinwirkung entsteht im lateralen Vorfußbereich ein Kippmoment von bis zu 4 mm. Dadurch wird das Großzehengrundgelenk entlastet und die Kraftübertragung auf die volle Breite des Vorfußes verteilt. Damit die Hebelwirkung beim Niederdrücken des gewölbten Bereichs unter dem Längsgewölbe realisiert werden kann, wird der Versteifungsbereich in einem festen und biegsamen Material ausgeführt, wie beispielsweise aus Carbon, vorzugsweise aus Carbonfaser, z.B. in Form einer Matrix aus Acrylharz oder Epoxidharz. Das hat den Vorteil, dass eine solche Schuheinlage thermoverformbar ist. Der Versteifungsbereich ist in einer bevorzugten Ausführungsform als Faserverbundteil ausgeführt. Zur Verstärkung und/oder Armierung der Matrix aus Polyester- oder Epoxidharz können Carbonfasern und/oder Glasfasern zum Einsatz kommen.

An dieser Stell sei klargestellt, dass gemäß der Erfindung die gesamt Schuheinlage von dem Versteifungsbereich gebildet sein kann, das heißt, dass die Schuheinlage lediglich oder im Wesentlichen aus dem Versteifungsbereich besteht. Gemäß der Erfindung ist allerdings auch denkbar, dass die Schuheinlage einen weich und elastisch ausgebildeten, nachgiebigen und insbesondere sich an den Fuß anschmiegsamen Sohlenbereich aufweist, der sich an den Versteifungsbereich anschließt oder auf dem der Versteifungsbereich aufgebracht ist und der eine im Wesentlichen der Fußkontur entsprechende Kontur aufweist.

Die Schuheinlage, insbesondere der Versteifungsbereich, bilden vorzugsweise eine geschwungene Raumform, die in unbelastetem Zustand hinter dem Längsgewölbe und im Bereich der Mittelfußköpfchen II bis V bzw. III bis V in der Nullebene liegt und im Bereich des Längsgewölbes gegen die Fußsohle gewölbt ist. Um durch die Schuheinlage möglichst wenig Raum im Schuh einzunehmen, ist die Schuheinlage in einer bevorzugten Ausführungsform von einheitlicher Dicke, vorzugsweise von einer Dicke von 1 mm bis 4 mm, besonders bevorzugt zwischen 2 mm bis 2,8 mm. Eine andere bevorzugte Ausführungsform sieht vor, dass die Schuheinlage im Zehenbereich eine Dicke von etwa 1,5 mm bis 2 mm, im Bereich des Großzehengrundgelenks und im Fersenbereich eine Dicke von etwa 2 mm bis 2,4 mm und an der lateralen Seite eine Dicke von 2 mm bis 4,2 mm aufweist. Diese Maßangabenbetreffen eine Situation, in der auf die Schuheinlage ein gewisser Druck, beispielsweise durch einen aufgelegten Fuß, ausgeübt wird und weiche Materialien wie Gewebe, Schaumstoff o.dgl. niedergedrückt sind.

Als vorteilhaft erweist es sich weiterhin, wenn sich der Versteifungsbereich bei aufgelegtem Fuß bis in den Fersenbereich erstreckt. Eine bevorzugte Ausführungsform sieht dabei vor, dass der Versteifungsbereich im mittleren Bereich der Ferse eine Aussparung aufweist. Seitlich der Aussparung setzt sich der Versteifungsbereich auf medialer und lateraler Seite in den Fersenbereich fort.

Eine weitere bevorzugte Ausführungsform der Erfindung sieht vor, dass die Schuheinlage sensomotorische Elemente u.a. eine Mittelfußpelotte und/oder Zehengreifer aufweist, welche zum einen sensomotorische Effekte und die Erhöhung des Tragekomforts zur Folge haben. Dabei sind die sensomotorische Elemente, die Mittelfußpelotte und/oder die Zehengreifer auf der Oberseite der Schuheinlage aufgebracht.

Eine andere bevorzugte Ausführungsform sieht vor, dass die Schuheinlage als Einlegesohle oder als orthopädische Einlage ausgebildet ist.

Die Erfindung betrifft weiter einen Schuh, in den die erfindungsgemäße Schuheinlage integriert ist, dessen Fußbett also beispielsweise in gleicher Weise ausgebildet ist, wie die erfindungsgemäße Schuheinlage, d.h. dass das Fußbett des Schuhs einen zusammenhängenden Versteifungsbereich aufweist, der zumindest einen Bereich der Schuheinlage umfasst, der sich bei aufliegendem Fuß im wesentlichen vom Längsgewölbe zum Vorfuß erstreckt, wobei der Versteifungsbereich bei aufliegendem Fuß im Bereich des Längsgewölbes gegen die Fußsohle gewölbt ist, im unbelasteten Zustand sich die Bereiche der Schuheinlage, die sich bei aufliegendem Fuß im Bereich des Rückfußes und des Vorfußes befinden, im wesentlichen in der Nullebene liegen und der Versteifungsbereich weiter so ausgebildet ist, dass bei aufgelegtem Fuß zumindest der Bereich des Großzehengrundgelenks außerhalb des Versteifungsbereichs angeordnet ist.

Die Erfindung wird nachfolgend unter Bezugnahme auf die Figuren der Zeichnungen an einem Ausführungsbeispiel näher erläutert. Es zeigen:
- Figur 1a, 1b: Veranschaulichung der Wirkung der Schuheinlage zur Vermeidung eines medialen Eindrehens des Fußes;
- Figur 2: eine schematische Darstellung einer beispielhaften Radsporteinlage in Draufsicht und
- Figur 3: eine schematische Darstellung einer beispielhaften Radsporteinlage in Seitenansicht (gesehen von der lateralen Seite).

Nachfolgend wir die Erfindung am Beispiel einer Radsporteinlage 100 näher erläutert. Dabei ist die Erfindung nicht auf Radsporteinlagen 100 eingeschränkt, sondern umfasst beispielsweise auch Einlegesohlen oder Schuhe mit einer integrierten Schuheinlage, solange sie nur die Merkmale der unabhängigen Ansprüche aufweisen.

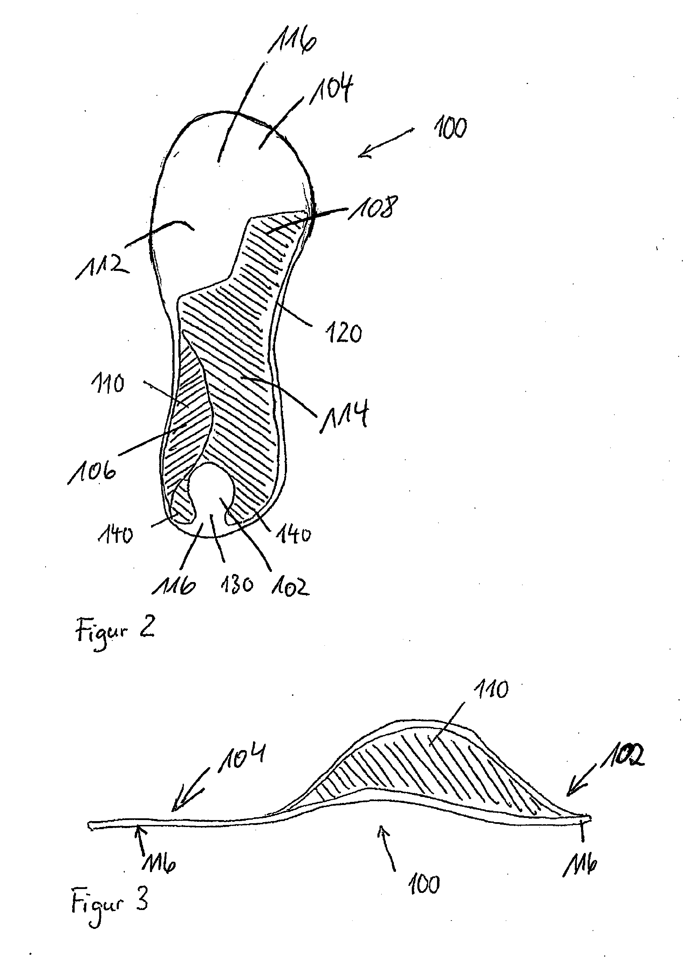

Die beispielhafte Radsporteinlage 100 verbleibt in unbelastetem Zustand im Rückfußbereich 102 und im Vorderfußbereich 104 in der Nullebene, insbesondere im Bereich hinter dem Längsgewölbe 106 und im Bereich der Mittelfußköpfchen 108. Der Rand der medialen Seite der Radsporteinlage 100 ist soweit hochgezogen, so dass das Längsgewölbe 106 des Fußes bzw. die knöcherne Struktur durch eine gegen die Fußsohle gewölbten Zone 110 der Einlage abgestützt wird. Das Großzehengrundgelenk (1. Strahl) liegt frei im Großzehengrundgelenkbereich 112 in der Radsporteinlage 100, ohne mit dem Versteifungsbereich 114, der in einer beispielhaften Ausführung als thermoplastisch carbonfaserverstärkter Acrylkern 120 ausgeführt ist, in Kontakt zu kommen. Der zusammenhängende thermoplastisch carbonfaserverstärkter Acrylkern 120 von der Ferse bis zum Vorfuß unterstützt und trägt den gesamten Fuß. Der Acrylkern 120 ist vorzugsweise nicht durch Quereinschnitte u.a. unterbrochen. Der Versteifungsbereich 114 wird beim gezeigten Ausführungsbeispiel vom Acrylkern 120 gebildet und ist in Fig. 1 und 2 schraffiert dargestellt. An den Versteifungsbereich 114 schließt sich insbesondere im Rückfußbereich 102 und im Vorderfußbereich 104 ein weich und elastisch ausgebildeter, nachgiebiger und sich an den Fuß anschmiegsamer Sohlenbereich 116 an. Entscheidend ist, dass der Versteifungsbereich 114 sich zwar nicht in den Großzehengrundgelenkbereich 112, sich aber bis in den lateralen Rands des Vorderfußbereichs 104, insbesondere bis in den Bereich unterhalb der Mittelfußköpfchen III bis V, erstreckt.

Es wäre auch denkbar die Einlage so auszubilden, dass sie lediglich aus dem Versteifungsbereich 114 besteht, der sich vom Bereich des Längsgewölbes 106 hin zu dem Bereich der lateralen Mittelfußköpfchen 108 erstreckt.

Beim Radfahren wird durch die Muskulatur über die Schien-und Wadenbeinknochen Kraft auf den Talus übertragen. Dadurch kommt es zum medialen Einknicken des Talus (Pronation), so dass die gegen die Fußsohle gewölbten Zone 110 niedergedrückt wird. Durch die besondere Form der Einlage 100, insbesondere da die Einlage einen zusammenhängenden carbonfaserverstärkten Acrylkern 120 vom Längsgewölbe bis zum Vorfuß aufweist, bewirkt der Druck auf die gegen die Fußsohle gewölbten Zone 110, dass die Einlage in eine Torsionsstellung gerät. In dieser Torsionsstellung ist der Fuß im Bereich des Vorfußes am lateralen Rand, je nach Steifigkeit des für den Versteifungsbereich verwendeten Materials und der Stärke der Krafteinwirkung, um bis zu ca. 4 mm angehoben. Durch die erfindungsgemäße Ausführung der Radsporteinlage 100 kann der Hebel durch das Niederdrücken der medialen gewölbten Zone 110 zusammen mit dem Bereich des carbonfaserverstärkten Acrylkerns 120 im Bereich des 3. bis 5. Strahls ungehindert wirken. Die Krafteinleitung vom Großzehengrundgelenk kann somit ungehindert auf den Schuh und somit auf das Pedal wirken.

Um diese Funktionalität zu erreichen, wird die Innenseite (mediale Seite) der Radsporteinlage 100 im Bereich der gewölbten Zone 110 soweit hochgezogen, dass die gewölbte Zone 110 das Längsgewölbe des Fußes bzw. die knöcherne Struktur abstützt und eine Hebelwirkung quer zum 5. Strahl erzeugt wird beim Niederdrücken der gewölbten Zone 110. Die Form der Radsporteinlage 100 bzw. des carbonfaserverstärkten Acrylkerns 120 ist so ausgestaltet, dass ein Druck im Rückfuß 102 die Radsporteinlage 100 im Bereich des Vorfußes 104 insbesondere im 3. bis 5. Strahl hoch hebelt. Dadurch wird die Kraftübertragung des gesamten Fußes auf die Schuhe/Pedal verbessert. Die genaue Ausgestaltung, d.h. wie hoch die Innenseite (mediale Seite) der Radsporteinlage 100 im Bereich der gewölbten Zone 110 gezogen und/oder bis unter welche Mittelfußköpfchen (III bis V oder II bis V) sich der Acrylkern 120 erstrecken muss, um das gewünschte Maß der Torsion bzw. Hebelwirkung zu erzielen, wird der Fachmann anhand seines Fachwissens in Anbetracht der gewünschten Wirkung erarbeiten.

Durch die Hebelwirkung, die insbesondere dadurch erreicht wird, dass der carbonfaserverstärkte Acrylkern 120 sich nicht bis unter das Großzehengrundgelenk erstreckt und die mediale Seite der gewölbten Zone 110 entsprechend hoch ausgeführt ist, ist es möglich, die gesamte Radsporteinlage 100 und insbesondere den Acrylkern 120 sehr dünn auszuführen. Eine beispielhafte Ausführungsform sieht vor, dass Radsporteinlage 100 /Acrylkern 120 gleichmäßig eine Dicke von 1,5 mm aufweisen, eine andere beispielhafte Ausführungsform sieht eine Dicke von 1,7 mm vor. Dadurch wird beim Tragen der Radsporteinlage 100 gegenüber herkömmlichen Einlagen im Schuh Platz gewonnen.

Eine beispielhafte Ausführungsform der Radsporteinlage 100 sieht vor, dass sich der carbonfaserverstärkte Acrylkern 120 bis in die Fersenregion erstreckt. Dabei weist der Acrylkern 120 im mittleren Fersenbereich eine Aussparung 130 auf. Durch zwei seitlich der Aussparung 130 sich erstreckende, nach hinten auslaufende Stabilisatoren 140 des Versteifungsbereichs 114 erhält die Radsporteinlage 100 in der Fersenregion die schalenartige Form. Dadurch geht der Acrylkern 120 der Bewegung des gesamten Fußes mit und gewährleistet, dass der Druck des Beins auf den Mittelfußbereich übertragen wird. Die Ausgestaltung des carbonfaserverstärkten Acrylkerns 120 im Fersenbereich beeinflusst die Hebelwirkung somit nicht negativ. Durch die schalige Form des Acrylkerns 120 im Rückfußbereich wird der gesamte Fuß geführt ohne die Kraftübertragung oder die Hebelwirkung zu beeinträchtigen.

Andere beispielhafte Ausführungsformen sehen eine zusätzliche individuelle Anpassung auf die Versorgung verschiedener Indikationen vor, beispielsweise durch gezieltes Aufbringen an bestimmte Punkten oder Bereiche auf der Oberseite der Radsporteinlage 100 von dünnen (max. bis 2 mm stark) Polsterteilen. Ebenso ist die Möglichkeit gewährleistet, den Tragekomfort zu erhöhen durch den Einsatz von Mittelfußpelotten und Zehengreifern.

Die gesamte Radsporteinlage 100 insbesondere durch den carbonfaserverstärkten Acrylkern 120, welcher federnd und flexibel ist, führt zu einer Vermeidung von Blutstau. Einem Einschlafen der Füße wird dadurch vorgebeugt.

Die Erfindung beschränkt sich in ihrer Ausführungsform nicht auf die vorstehend angegebenen bevorzugten Ausführungsbeispiele. Vielmehr ist eine Anzahl von Varianten denkbar, die von der erfindungsgemäßen Anordnung auch bei grundsätzlich anders gearteten Ausführungen Gebrauch macht.

## Patentansprüche

1. Sport-Schuheinlage (100) zur Verbesserung der Kraftübertragung, die einen zusammenhängenden Versteifungsbereich (114) aufweist, der zumindest einen Bereich der Schuheinlage (100) umfasst, der sich bei aufliegendem Fuß vom Längsgewölbe (106) zum Vorfuß (104) erstreckt, wobei der Versteifungsbereich (114) bei aufliegendem Fuß im Bereich des Längsgewölbes (106) gegen die Fußsohle gewölbt ist, im unbelasteten Zustand sich die Bereiche der Schuheinlage (100), die sich bei aufliegendem Fuß im Bereich des Rückfußes (102) und des Vorfußes (104) befinden, in der Nullebene liegen, wobei der Versteifungsbereich (120) zur Realisierung einer Hebelwirkung in einem festen und biegsamen Material ausgebildet ist,
**dadurch gekennzeichnet, dass** der Versteifungsbereich (114) so ausgebildet ist, dass bei aufgelegtem Fuß zumindest der Bereich des Großzehengrundgelenks (112) außerhalb des Versteifungsbereichs (114) und der Bereich der lateralen Mittelfußköpfe (108) innerhalb des Versteifungsbereichs (114) angeordnet ist,
so dass bei aufgelegtem Fuß und einer Pronation des Fußes der gewölbte Bereich (110) unter dem Längsgewölbe niedergedrückt wird und dies zu einer Anhebung des Versteifungsbereichs (114) unter dem Vorfuß (104) führt, wobei der mediale Mittelfußkopf entlastet und die lateralen Mittelfußköpfe zur gleichmäßigeren Kraftverteilung belastet werden.

2. Schuheinlage (100) nach Anspruch 1, **dadurch gekennzeichnet, dass** zumindest der Versteifungsbereich (114) als eine geschwungene Raumform von im Wesentlichen gleicher Dicke ausgebildet ist.

3. Schuheinlage (100) nach einem der vorherigen Ansprüche, **dadurch gekennzeichnet, dass** sich der Versteifungsbereich (114) bei aufgelegtem Fuß bis unter die Mittelfußköpfchen III bis V erstreckt.

4. Schuheinlage (100) nach einem der voranstehenden Ansprüche, **dadurch gekennzeichnet, dass** der Versteifungsbereich (114) m Bereich des Rückfußes am medialen und lateralen Rand in zwei Stabilisatoren (140) ausläuft.

5. Schuheinlage (100) nach Anspruch 4, **dadurch gekennzeichnet, dass** der Versteifungsbereich (114) im Fersenbereich zwischen den Stabilisatoren eine Aussparung aufweist.

6. Schuheinlage (100) nach einem der voranstehenden Ansprüche, **dadurch gekennzeichnet, dass** der Versteifungsbereich als eine Matrix aus Polyester- oder Epoxidharz ausgebildet ist.

7. Schuheinlage (100) nach Anspruch 6, **dadurch gekennzeichnet, dass** die Matrix durch Carbonfasern und/oder Glasfasern verstärkt ist.

8. Schuheinlage (100) nach einem der voranstehenden Ansprüche, **dadurch gekennzeichnet, dass** die Schuheinlage Zehengreifer und/oder sensomotorische Elemente aufweist.

9. Schuheinlage (100) nach einem der voranstehenden Ansprüche, **dadurch gekennzeichnet, dass** die Schuheinlage als orthopädische Einlage oder als Einlegesohle ausgebildet ist.

10. Sportschuh, bei dem das Fußbett einen zusammenhängenden Versteifungsbereich aufweist, der zumindest einen Bereich der Schuheinlage umfasst, der sich bei aufliegendem Fuß vom Längsgewölbe zum Vorfuß erstreckt, wobei der Versteifungsbereich bei aufliegendem Fuß im Bereich des Längsgewölbes gegen die Fußsohle gewölbt ist, im unbelasteten Zustand sich die Bereiche der Schuheinlage, die sich bei aufliegendem Fuß im Bereich des Rückfußes und des Vorfußes befinden, in der Nullebene liegen und der Versteifungsbereich weiter so ausgebildet ist, wobei der Versteifungsbereich zur Realisierung einer Hebelwirkung in einem festen und biegsamen Material ausgebildet ist, **dadurch gekennzeichnet, dass** bei aufgelegtem Fuß zumindest der Bereich des Großzehengrundgelenks außerhalb des Versteifungsbereichs angeordnet ist und der Bereich der lateralen Mittelfußköpfe (108) innerhalb des Versteifungsbereichs (114) angeordnet ist so dass bei aufgelegtem Fuß und einer Pronation des Fußes der gewölbte Bereich (110) unter dem Längsgewölbe niedergedrückt wird und dies zu einer Anhebung des Versteifungsbereichs (114) unter dem Vorfuß (104) führt, wobei der mediale Mittelfußkopf entlastet und die lateralen Mittelfußköpfe zur gleichmäßigeren Kraftverteilung belastet werden.

## Claims

1. Sports shoe insert (100) for improving force transmission, comprising a cohesive reinforcement region (114) which covers at least one area of the shoe insert (100) which when stood upon extends from the longitudinal arch (106) to the forefoot (104), wherein when stood upon, the reinforcement region (114) is curved against the foot sole in the area of the longitudinal arch (106), in unloaded state the areas of the shoe insert (100), which lie in the region of the hindfoot (102) and the forefoot (104) when stood upon, lie in the neutral plane, wherein the reinforcement region (120) is formed from a solid and flexible material to achieve a lever effect, **characterized in that** the reinforcement region (114) is formed such that when stood upon, at least the area of the big toe base joint (112) is arranged outside the reinforcement region (114) and the area of the lateral metatarsal bone heads (108) is arranged inside the reinforcement region (114), so that when stood upon and under a pronation of the foot, the curved area (110) below the longitudinal arch is pressed down and this leads to a lifting of the reinforcement region (114) below the forefoot (104), relieving the load on the medial metatarsal bone head and loading the lateral metatarsal bone heads for a more even force distribution.

2. Shoe insert (100) according to claim 1, **characterized in that** at least the reinforcement region (114) is formed as a curved three-dimensional shape of substantially constant thickness.

3. Shoe insert (100) according to any of the preceding claims, **characterized in that** when stood upon, the reinforcement region (114) extends to below the metatarsal bone heads III to V.

4. Shoe insert (100) according to any of the preceding claims, **characterized in that** the reinforcement region (114) in the area of the hindfoot runs out into two stabilisers (140) at the medial and lateral edges.

5. Shoe insert (100) according to claim 4, **characterized in that** the reinforcement region (114) has a recess between the stabilisers in the heel area.

6. Shoe insert (100) according to any of the preceding claims, **characterized in that** the reinforcement region is formed as a matrix of polyester or epoxy resin.

7. Shoe insert (100) according to claim 6, **characterized in that** the matrix is reinforced with carbon fibres and/or glass fibres.

8. Shoe insert (100) according to any of the preceding claims, **characterized in that** the shoe insert has toe grippers and/or senso-motor elements.

9. Shoe insert (100) according to any of the preceding claims, **characterized in that** the shoe insert is formed as an orthopaedic insert or as an in-sole.

10. Sports shoe in which the foot bed comprises a cohesive reinforcement region which covers at least one area of the shoe insert which, when stood upon, extends from the longitudinal arch to the forefoot, wherein when stood upon, the reinforcement region is curved against the foot sole in the area of the longitudinal arch, in unloaded state the regions of the shoe insert, which lie in the region of the hindfoot and the forefoot when stood upon, lie in the neutral plane, and the reinforcement region is furthermore formed from a solid and flexible material to achieve a lever effect, **characterized in that** when stood upon, at least the area of the big toe base joint is arranged outside the reinforcement region and the area of the lateral metatarsal bone heads (108) is arranged inside the reinforcement region (114), so that when stood upon and under a pronation of the foot, the curved region (110) below the longitudinal arch is pressed down and this leads to a lifting of the reinforcement region (114) below the forefoot (104), relieving the load on the medial metatarsal bone head and loading the lateral metatarsal bone heads for a more even force distribution.

## Revendications

1. Semelle intérieure de chaussure de sport (100) destinée à améliorer la transmission de puissance, qui présente une partie de raidissement associée (114) qui couvre au moins une partie de la semelle intérieure de chaussure (100) qui s'étend, lorsque le pied est en appui, de la voûte plantaire (106) jusqu'à l'avant-pied (104), dans laquelle la partie de raidissement (114) est bombée, lorsque le pied est en appui, dans la zone de la voûte plantaire (106) contre la plante du pied, les zones de la semelle intérieure de chaussure (100), qui se trouvent dans la zone de l'arrière-pied (102) et de l'avant-pied (104) lorsque le pied n'est pas en appui, sont étendues dans le plan de référence zéro à l'état non chargé, dans laquelle la partie de raidissement (120) est réalisée en un matériau solide et souple pour fournir un effet de levier,
**caractérisé en ce que** la partie de raidissement (114) est formée de telle sorte que lorsque le pied est en appui, au moins la zone de l'articulation du gros orteil (112) est disposée à l'extérieur de la partie de raidissement (114), et la zone des têtes de métatarse latérales (108) est disposée à l'intérieur de la partie de raidissement (114), de sorte qu'avec le pied en appui et une pronation du pied, la zone bombée (110) située sous la voûte plantaire va être enfoncée, ceci conduisant à une augmentation de la partie de raidissement (114) jusque sous l'avant-pied (104), de sorte que la tête de métatarse médiane va être déchargée et les têtes de métatarse latérales vont être chargées, pour une répartition des forces plus uniforme.

2. Semelle intérieure de chaussure (100) selon la revendication 1, **caractérisée en ce qu'**au moins la partie de raidissement (114) est réalisée sous la forme d'une forme tridimensionnelle sinueuse d'une épaisseur sensiblement identique.

3. Semelle intérieure de chaussure (100) selon l'une quelconque des revendications précédentes, **caractérisée en ce que** la partie de raidissement (114) s'étend, avec le pied en appui, jusque sous les têtes des métatarses III à V.

4. Semelle intérieure de chaussure (100) selon l'une quelconque des revendications précédentes, **caractérisée en ce que** la partie de raidissement (114) se termine, dans la zone de l'arrière-pied, par deux stabilisateurs (140) sur les bords médial et latéral.

5. Semelle intérieure de chaussure (100) selon la revendication 4, **caractérisée en ce que** la partie de raidissement (114) présente un évidement dans la région du talon entre les stabilisateurs.

6. Semelle intérieure de chaussure (100) selon l'une quelconque des revendications précédentes, **caractérisée en ce que** la partie de raidissement est réalisée sous la forme d'une matrice de polyester ou de résine époxy.

7. Semelle intérieure de chaussure (100) selon la revendication 6, **caractérisée en ce que** la matrice est renforcée par des fibres de carbone et/ou des fibres de verre.

8. Semelle intérieure de chaussure (100) selon l'une quelconque des revendications précédentes, **caractérisée en ce que** la semelle intérieure de chaussure comporte des éléments de préhension d'orteil et/ou des éléments sensomoteurs.

9. Semelle intérieure (100) selon l'une quelconque des revendications précédentes, **caractérisée en ce que** la semelle intérieure de chaussure est réalisée sous la forme d'une semelle orthopédique ou d'une semelle première.

10. Chaussure de sport, dans laquelle la semelle présente une partie de raidissement associée qui couvre au moins une partie de la semelle intérieure de chaussure qui s'étend, lorsque le pied est en appui, de la voûte plantaire jusqu'à l'avant-pied, dans laquelle la partie de raidissement est bombée, lorsque le pied est en appui, dans la zone de la voûte plantaire contre la plante du pied, les zones de la semelle intérieure de chaussure, qui se trouvent dans la zone de l'arrière-pied et de l'avant-pied lorsque le pied n'est pas en appui, sont étendues dans le plan de référence zéro à l'état non chargé, dans laquelle la partie de raidissement est réalisée en un matériau solide et souple pour fournir un effet de levier, **caractérisé en ce que** lorsque le pied est en appui, au moins la zone de l'articulation du gros orteil est disposée à l'extérieur de la partie de raidissement, et la zone des têtes de métatarse latérales (108) est disposée à l'intérieur de la partie de raidissement (114), de sorte qu'avec le pied en appui et une pronation du pied, la zone bombée (110) située sous la voûte plantaire va être enfoncée, ceci conduisant à une augmentation de la partie de raidissement (114) jusque sous l'avant-pied (104), de sorte que la tête de métatarse médiane va être déchargée et les têtes de métatarse latérales vont être chargées, pour une répartition des forces plus uniforme.
